# EUROPEAN PATENT SPECIFICATION

(11) **EP 2 263 550 B1**
(45) Date of publication and mention of the grant of the patent: **15.07.2015**
(21) Application number: 10166375.5
(22) Date of filing: 17.06.2010
(51) Int. Cl.: A61B 10/02

(54) **Telescoping biopsy device**
TELESKOPIERBARE BIOPSIEVORRICHTUNG
DISPOSITIF DE BIOPSIE TÉLESCOPIQUE

(30) Priority: 18.06.2009 US 218312 P; 28.05.2010 US 790266
(43) Date of publication of application: 22.12.2010
(73) Proprietor: Cook Medical Technologies LLC, Bloomington, IN 47404 (US)
(72) Inventor: PENDLETON, Steven L, Spencer, IN Indiana 47460 (US)
(74) Representative: Garratt, Peter Douglas

(56) References cited:
- WO-A1-96/22056
- WO-A2-2009/009274
- US-A- 5 573 008
- US-A- 5 871 453
- US-A1- 2002 049 442

## Description

### CROSS-REFERENCE TO RELATED APPLICATIONS

### TECHNICAL FIELD

This disclosure relates to devices configured to obtain biopsy samples from within a patient. It is known to provide devices configured to extend within a patient, such as opposing forceps, needles, brushes, and the like. Such devices are normally used for only a single sample and then must be withdrawn to retrieve the sample. Additionally, the various conventional biopsy devices are not configured for removing biopsy samples at varying depths. Further, after a biopsy sample is removed from a patient, the tissue often bleeds which must be controlled or managed by withdrawing the biopsy device and inserting various tools to eliminate bleed. This multiple tool procedure becomes time consuming, and therefore inefficient and costly in situations where multiple biopsy samples are needed from a patient.
Reference is directed to US Patent 5,573,008 which discloses an instrument for taking multiple biopsy samples without being withdrawn from the body. An axially movable inner sample tube moves together with an outer axially moveable jaw closing sleeve to open and close a pair of jaws. Reference is also directed to US published patent application 2002/0049442 which discloses a biopsy sample with a cutter near its distal end for resecting and containing a tissue sample and a sheath which includes an electrode element for cauterizing tissue.

### BRIEF SUMMARY

A first representative embodiment of a biopsy device is provided. The biopsy device includes a first elongate substantially tubular member that defines a distal end portion and a proximal end portion with a lumen defined therethrough. A second elongate substantially tubular member is telescopically and slidably disposed within the lumen of the first member, the second member comprising a distal end portion and a proximal portion with a lumen defined therethrough. The distal end portion of the second member comprises two or more metallic jaws that are biased toward each other. A third elongate tubular member is telescopically and slidably received within the lumen of the second member. The third member includes a distal end portion with an open distal end, configured to allow foreign material to enter the lumen therefrom. The distal end portion of the third portion is configured to maintain the jaws in a separated configured when the jaws surround the distal end portion of the third tubular member. In some embodiments, the second member is rotatable with respect to the third member.

In some representative embodiments the device includes a handle receiving the proximal end portions of each of the first, second, and third members. The handle comprises a first operator configured to translate the second member with respect to the first member, and a second operator configured to translate the third member with respect to the second member. The first and second operators may be removably coupled together to provide for simultaneous and consistent motion of the second and third members with respect to the first member.

In some representative embodiments, the energy source is a battery disposed within the device.

In some representative embodiments, an energy transmission line is enclosed by the proximal portion of the first member to provide a flow of electrical current to the jaws from an energy source.

In some representative embodiments, the distal end portions of the first and third members are formed from substantially electrically insulating materials.

In some representative embodiments, the device includes an interlock preventing application of energy to the jaws unless the distal end portion of the third member is at least partially telescopically withdrawn proximally of the jaws.

In some representative embodiments the distal end portion of the second member comprises a tube of superelastic material formed from a sheet, the jaws are separated by longitudinal slots blindly defined along a portion of the length of the tube from the distal end.

In some representative embodiments, the second member comprises a flexible portion that is fixed to the jaws.

In some representative embodiments a detent is defined upon one of the second and third members and a plurality of recesses are defined along the length of the other of the second and third members, the detent is engageable with each of the plurality of recesses to maintain the second and third members longitudinally and rotationally fixed together at a plurality of different mutual orientations.

In some representative embodiments, the jaws each comprise a sharpened edge configured to cut tissue when pressed thereon.

In some representative embodiments the third member is configured to receive a source of suction through its lumen to urge foreign material proximally through the lumen.

In some representative embodiments, a filter is disposed in communication with the lumen of the third member.

In some representative embodiments the combined distal end portions of the first, second and third members are sufficiently flexible to be aligned at an oblique angle to a longitudinal axis through the proximal end portion of the first member.

In some representative embodiments the distal end portion of the third member is configured to retain a tissue sample removed by the jaws.

A second representative embodiment of a biopsy device is provided. The device includes a first relatively flexible tubular member with a distal end portion and a proximal end portion and a lumen defined therethrough. A second relatively flexible tubular member is slidably mounted within the lumen of the first tubular member. The second member comprises a distal end portion, a proximal end portion, and a lumen defined therethrough, wherein the distal end portion comprises a plurality of metallic jaws that are biased to a closed position where edges of each of the jaws are aligned in close proximity or in contact with each other. A third relatively flexible tubular member is slidably mounted within the lumen of the second member, the third member comprising a distal end portion, a proximal end portion and a lumen therethrough, the distal end portion configured to maintain the jaws of the second member in a substantially tubular orientation when the distal end portion of the third member is aligned between the jaws.

In some representative embodiments the device includes a handle receiving the proximal end portions of each of the first, second, and third members. The handle comprises a first operator configured to translate the second member with respect to the first member, and a second operator configured to translate the third member with respect to the second member. The first and second operators may be removably coupled together to provide for simultaneous and consistent motion of ht second and third members with respect to the first member.

In some representative embodiments, the distal end portions of the first and third members are formed from substantially electrically insulating materials.

In some representative embodiments, the device includes an interlock preventing application of energy to the jaws unless the distal end portion of the third member is at least partially telescopically withdrawn proximally of the jaws.

In some representative embodiments the distal end portion of the second member comprises a tube of superelastic material formed from a sheet, the jaws are separated by longitudinal slots blindly defined along a portion of the length of the tube from the distal end.

In some representative embodiments, the second member comprises a flexible portion that is fixed to the jaws.

In some representative embodiments a detent is defined upon one of the second and third members and a plurality of recesses are defined along the length of the other of the second and third members, the detent is engageable with each of the plurality of recesses to maintain the second and third members longitudinally and rotationally fixed together at a plurality of different mutual orientations.

In some representative embodiments, the jaws each comprise a sharpened edge configured to cut tissue when pressed thereon.

In some representative embodiments the third member is configured to receive a source of suction through its lumen to urge foreign material proximally through the lumen.

In some representative embodiments, a filter is disposed in communication with the lumen of the third member.

In some representative embodiments the combined distal end portions of the first, second and third members are sufficiently flexible to be aligned at an oblique angle to a longitudinal axis through the proximal end portion of the first member.

In some representative embodiments the distal end portion of the third member is configured to retain a tissue sample removed by the jaws.

A third representative embodiment of a biopsy device is provided. The device includes a first relatively flexible tubular member with a distal end portion, a proximal end portion, and a lumen defined therethrough. The distal end portion comprises a plurality of metallic jaws that are biased to a closed position where an edge of each of the jaws are aligned in close proximity or in contact to each other. A second relatively flexible tubular member is slidably mounted within the lumen of the first member, the second member comprising a distal end portion, a proximal end portion and a lumen therethrough, the distal end portion configured to maintain the jaws in a retracted orientation when the distal end portion of the second member is aligned between the jaws. The jaws are configured to receive energy from a remote source to selectively cauterize tissue proximate to the jaws.

Another representative embodiment of a biopsy device is provided. The device includes an outer tubular member, an intermediate tubular member, and an inner tubular member each movably and telescopically connected together. The intermediate and central members being slidably disposed within and with respect to the outer member, and the central member being slidably disposed within and with respect to the intermediate member. The intermediate member includes a plurality of flexible jaws that are maintained in a generally tubular orientation when the central member is disposed therethrough, and biasingly transform to a closed orientation when the central member is withdrawn from between the jaws. The jaws are configured to receive a source of flowing energy, such as electrical current, to dramatically increase the temperature of the jaws.

Another representative embodiment of a biopsy device is provided. The device includes an outer tubular member and an inner tubular member telescopically disposed within a lumen of the outer tubular member. The outer member comprises a plurality of jaws biased toward a closed configuration, that pivot to a substantially tubular open configuration when the inner member is disposed therebetween. The device further includes a handle that is fixed to one of the outer and inner members and includes an operator fixed to the other of the outer and inner members to cause that member to translate with respect to the member fixed to the handle. The jaws are configured to receive an energy source, such as electrical current or RF energy and obtain a significantly elevated temperature upon receipt of energy thereon. The device 10 may include an interlock that prevents application of energy to the jaws when the central member is disposed between the plurality of jaws.

A method for removing a biopsy sample is provided. The method includes the steps of positioning a device proximate to tissue to be removed, the device including a first tubular member, a second tubular member, and a third tubular member. The second tubular member includes two or more jaws disposed upon a distal portion thereof. The second tubular member is telescopically disposed and moveable within a lumen defined within the first member, and a third tubular member is movably disposed within a lumen of the second member. The method further includes the steps of penetrating the jaws into tissue and withdrawing the third member proximally with respect to the second member to allow the jaws to pivot toward each other to cut the tissue disposed between the jaws from neighboring tissue. The method additionally includes the step of applying energy to the jaws to cauterize remaining tissue within the patient.

Advantages of the present disclosure will become more apparent to those skilled in the art from the following description of the preferred embodiments of the disclosure that have been shown and described by way of illustration. As will be realized, the disclosed subject matter is capable of other and different embodiments, and its details are capable of modification in various respects. Accordingly, the drawings and description are to be regarded as illustrative in nature and not as restrictive.

### BRIEF DESCRIPTION OF THE DRAWINGS

FIG. 1 is a perspective view of a biopsy device in a first configuration.

FIG. 2 is the view of FIG. 1 with the device in a second configuration with the intermediate and central members extended from the outer member.

FIG. 3 is the view of FIG. 1 in a third configuration with the jaws of the intermediate member extended past the central member.

FIG. 4 is a perspective view of the device of FIG. 1 with the handle removed.

FIG. 5 is a detail cross-sectional view of the device of FIG. 1 with the distal end portion of the device contacting tissue for removal of a biopsy sample.

FIG. 6 is the view of FIG. 5 showing the jaws and the central member extending into tissue.

FIG. 7 is the view of FIG. 5 showing the central member retracted from the jaws that are biased into the closed position with a biopsy sample removed therebetween.

FIG. 8 is the view of FIG. 5 showing the central member further withdrawn proximally and the biopsy sample withdrawn from the jaws.

FIG. 9 is the view of FIG. 5 showing the jaws withdrawn from the tissue.

FIG. 10 is a detail cross-sectional view of the device of FIG. 8 showing an energy transmission line disposed in conjunction with the intermediate member and jaws, with the jaws energized to cauterize the remaining tissue.

FIG. 11 is a detail view of the jaws of FIG. 1 in an open configuration.

FIG. 12 is the view of FIG. 11, showing the jaws in the closed configuration.

FIG. 12a is a front view of the jaws of FIG. 11, showing the jaws in the closed configuration.

FIG. 13 is a detail view of an alternate set of jaws in the open position.

FIG. 14 is the view of FIG. 13 showing the jaws in the closed position.

FIG. 14a is a front view of the jaws of FIG. 13, showing the jaws in the closed configuration.

FIG. 15 is a perspective view of the central member of the device of FIG. 1.

FIG. 16 is a perspective view of an alternate central member.

FIG. 17 is a perspective view of an intermediate member that is configured to be used with the central member of FIG. 16.

FIG. 18 is a perspective view of another biopsy device in a first configuration.

FIG. 19 is the view of FIG. 18 in a second configuration with the jaws extended from the inner member.

FIG. 20 is a perspective view of another biopsy device in a normal configuration.

FIG. 21 is the view of FIG. 21 with the inner member extend outward from the sheath.

FIG. 22 is a perspective view of the inner member in a normal configuration.

FIG. 23 is the view of the inner member of FIG. 22 with the fingers in a compressed configuration.

FIG. 24 is a cross-sectional view of the distal portion of the device of FIG. 20.

FIG. 25 is a cross-sectional view of the distal portion of the device of FIG. 21.

### DETAILED DESCRIPTION OF THE DRAWINGS AND THE PREFERRED EMBODIMENTS

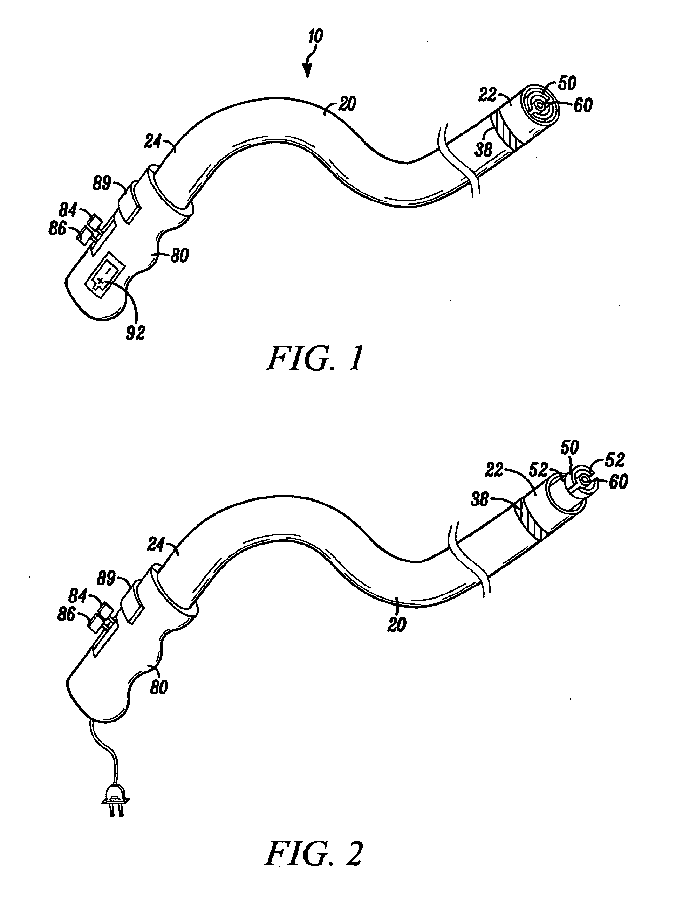

Turning now to FIGs. 1-3, a biopsy device 10 configured to remove a sample of internal tissue from a human or mammal is provided. The device 10 includes an outer tubular member 20, an intermediate tubular member 40 disposed within a lumen 21 of the outer member 20, and a central tubular member 60 disposed within a lumen 41 of the intermediate member 40. Each of the outer, intermediate, and central members 20, 40, 60 may be catheters, cannulas, sheaths, or other tubular members that are configured to operate as described herein.

The intermediate member 40 is telescopically and slidably disposed within the lumen 21 of the outer member 20. The central member 60 is telescopically slidably disposed within the lumen 41 of the intermediate member 40 and can be freely translated with respect to each of the intermediate member 40 and the outer member 20. The device 10 is configured to remove tissue accessible through a lumen defined with a patient, such as tissue accessible through the patient's urethra, ureter, or other similar urological locations. The device 10 may be used in conjunction with a endoscope, ureteroscope, cystoscope, or the like or with an access sheath such as the Flexor® sold by Cook Urological, Inc. Similarly, the device may be appropriate for use in the patients GI or biliary system used in conjunction with an endoscope. The device 10 may be also or alternatively used to obtain tissue samples from locations in a patient accessed percutaneously. The device 10 may come in various lengths suitable for various medical procedures and various portions of the patient's anatomy. For example, the device may be in lengths of 65 cm, 120 cm, or other suitable lengths. In these situations, the outer tubular member 20 is generally the rated length with the intermediate and central members 40, 60 disposed therethrough of longer lengths than the outer member 20 to allow each member to be separately manipulated from the proximal end of the device.

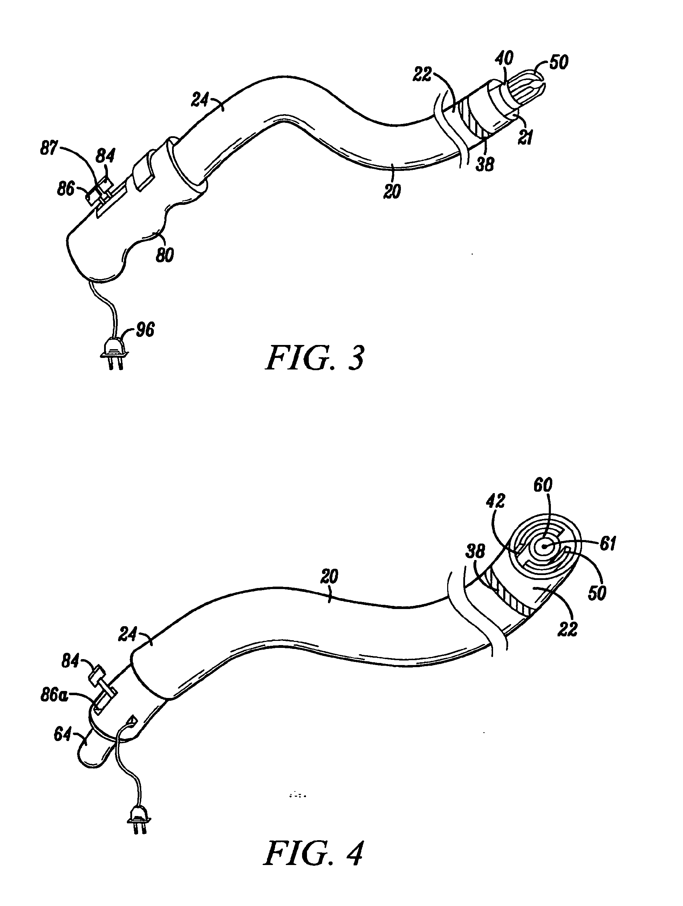

The intermediate member 40 includes a distal end portion 41 and a proximal end portion 44, with the lumen 41 defined therethrough. The intermediate member 40 may be longer than the outer member 20, such that the position of the intermediate member 40 may be easily adjusted with respect to the outer member 20 when the device 10 is inserted into the patient and the distal end portion 42 and jaws 50 of the intermediate member 40 aligned with (or just inside of) the distal end portion of the outer member. The distal end portion 42 includes a plurality of jaws 50 that are disposed upon an end of the distal end portion 42. The jaws 50 are biased into a closed position (FIG. 3) such that the tips or edges 52 of the jaws 50 are disposed either in contact with each other, or in close physical proximity to each other. The plurality of jaws 50 maybe flexed outward from the closed position and maintained in an open position (FIG. 2) against the inward biasing force when the distal end portion 62 of the central member 60 is disposed between the plurality of jaws 50.

The plurality of jaws 50 may be a part of or may extend from an end of the distal end portion 42. In some embodiments, the proximal end portion 44 and the distal end portion 42 (exclusive of the plurality of jaws 50) may be made from a suitable flexible material with adequate hoop strength to cause the intermediate member 40 to retain the integrity of the lumen 41 as the device 10 is passed through relative tortuous paths within the patient, such as when the device is passed through the urethra or ureter, or through various occluded or partially occluded paths within the patient. In some embodiments, the intermediate member 40 may be polyurethane, nylon, polyethylene, PEEK, or a variety of other materials. In embodiments where the distal end portion 42 is made from a differing material than the plurality of jaws 50, the jaws may be fixed to the end of the distal end portion 42, with adhesive, a press fit connection, fasteners, or with other connection members or devices known in the art. In some embodiments, one or both of the inner and outer surfaces of the intermediate member 40 may be coated with a relatively lubricious coating to allow sliding movement between the intermediate member and the contacting outer and central members 20, 60.

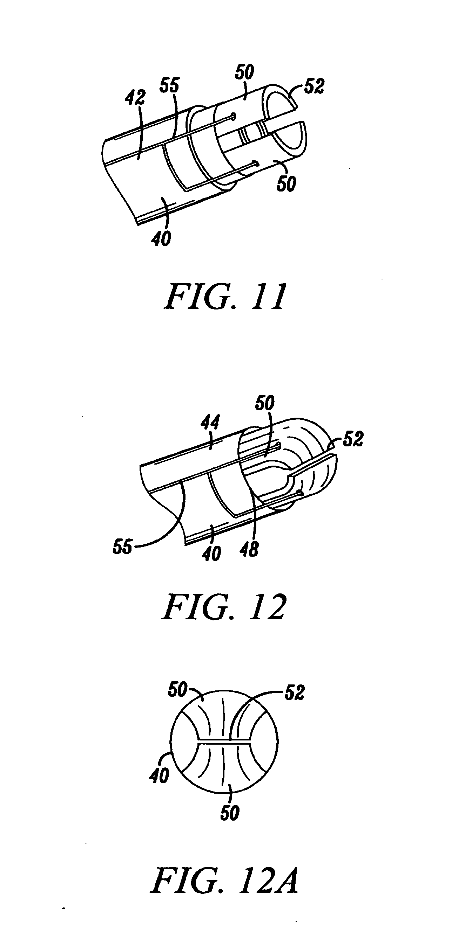

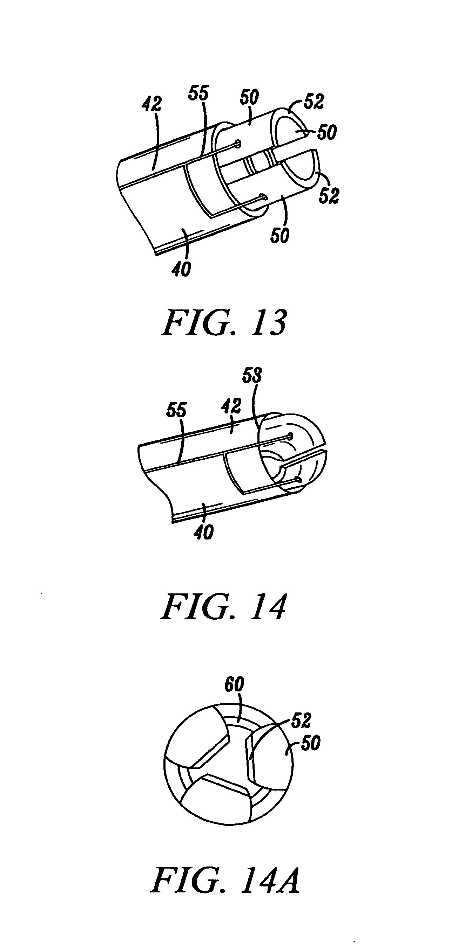

The plurality of jaws 50 may manufactured from a superelastic material, such as Nitinol or various alloys thereof. In other embodiments, the plurality of jaws 50 may be manufactured from stainless steel, or other medically acceptable materials. The plurality of jaws 50 may include two opposed jaws (FIGs. 11-12a), three opposed jaws (FIGs. 13-14a), four opposed jaws, or another number of opposed jaws. The plurality of jaws 50 may be made from a length of tubular Nitinol (or a sheet of flat Nitinol formed into a tube and welded or otherwise fixed together). The plurality of jaws 50 may be formed by machining a plurality of slits from an end of the tube blindly along a portion of the length of the tube (corresponding to the desired length of the jaws), with the number of slits equal to the number of desired jaws. The slits are normally made at constant intervals to form each jaw with a similar size, while in other embodiments the slits may be made at different spacing to form jaws 50 of different widths as desired.

The jaws 50 are preferably formed or "trained" to be biased into a normal compressed orientation (FIG. 1) where the ends 52 of the various jaws 50 contact the ends of the other opposed jaws 50, or in other embodiments, the ends 52 of the various jaws 50 are positioned in close proximity to the ends 52 of the opposed jaws 50. In some embodiments, only a distal portion of each of the plurality of jaws 50 are disposed in an angled fashion toward the other opposed jaws 50 (FIG. 3), while a proximal portion of each of the plurality of jaws 50 collectively form a generally tubular profile. In embodiments where only a distal portion of each of the plurality of jaws are aligned in an orientation toward the remainder of the jaws 50 and the proximal portion of each of the jaws collectively form a tubular profile, the jaws 50 may include a discrete edge 53 (shown in FIG. 14) between the distal and proximal portions, while in other embodiments, the distal and proximal portions of the jaws 50 may be separated with a gradual continuous curve in the material forming each of the plurality of jaws 50.

The plurality of jaws 50 may be urged to a second open position (FIG. 2) where the plurality of jaws 50 are maintained away from each other, with the plurality of jaws 50 collectively forming a general tubular profile with a consistently sized outer diameter and a lumen with substantially the same inner diameter as the remainder of the intermediate member 40. The plurality of jaws 50 may be "trained" into the closed position when in the austenite phase using conventional methods of training Nitinol, various alloys of Nitinol, or other superelastic materials, as known in the art.

The plurality of jaws 50 may each include a sharpened edge defined upon the distal end hereof, such as a beveled edge, a "v" shaped edge, etc. The distal edge of each of the plurality of jaws 50 may be aligned along a curve (similar to the curve that defines the tube formed collectively by the proximal ends of the plurality of jaws 50. In other embodiments, the edges of the plurality of jaws 50 may be straight, waved, stepped, corrugated, or in other shape or alignment. As discussed above the edge of each to the plurality of jaws 50 are formed such that the jaws collectively form a tube with generally the same outer diameter and inner diameter as the remainder of the intermediate member.

The plurality of jaws 50 are each configured to receive a source of energy from a remote source thereof, such as electrical current or RF energy, or the like (referred to as electrical current hereafter for the sake of brevity). Upon selective receipt of energy upon the jaws 50, the jaws 50 heat to an elevated temperature suitable to cauterize or burn tissue contacting or proximate the jaws 50. As discussed herein, tissue often bleeds after a biopsy sample or core is withdrawn therefrom and the heated jaws cauterize the remaining tissue preventing further blood loss therefrom. One or more wires 55 may be disposed in, through, or in conjunction with the proximal and distal end portions 42, 44 of the intermediate member 40 to allow electrical current (a flow of RF energy, or the like) to flow to the plurality of jaws 50 from the remote source of energy. In some embodiments, the wires 55 are disposed within the lumen 41 of the intermediate member 40 or upon the outer surface of the intermediate member 40. In further embodiments, the intermediate member may be extruded or otherwise formed with the wires 55 passing through the walls of the intermediate member 40. The handle 80 of the device 10 may include a switch, button, or other control device 89 to allow electrical current or the like to selectively flow to the plurality of jaws 50. The jaws 50 accordingly heat up with the application of electrical or other energy to a suitable temperature to cauterize or burn tissue that contacts the jaws 50. Suitable devices will allow for fairly rapid heat up rates of the jaws 50 with a minimal application of electrical energy therethrough, both to minimize the time necessary to cauterize the patient's tissue and to minimize the power draw of the device and the current flowing within the patient.

The outer member 20 may be an elongate tubular member that defines a lumen that retains both the intermediate member 40 and the central member 60 therethrough. The lumen 21 includes a inner diameter substantially the same as the outer diameter of the intermediate member (or only slightly greater than) such that the intermediate member 40 is free to telescopically move with respect to the outer member 20, but maintain a majority of surface to surface contact between the outer and intermediate members 20, 40 to prevent spurious undesired relative telescoping motion. The outer diameter of the outer member 20 may be various sizes to accommodate various medical situations, portions of the patient's anatomy, and various types of endoscopes or the like. In urological embodiments, the outer diameter of the outer member may be 5.2 Fr or less, such as 5.0, 4.5, 4.0 or 3.5 Fr, which allow the device 10 to be passed through lumens of typical sheaths (such as the Flexor® Ureteral Access Sheath, sold by Cook Urological, Inc.) or working channels of scopes that have lumens of 9.5, 12.0 Fr. or similar inner diameters. Providing an outer diameter of the device 10 of no larger than 5.2 Fr. allows other components or fluid to be passed through the sheath or scope in parallel with the device 10.

The distal end 22 of the outer member 20 is preferably soft enough to prevent the outer member 20 from cutting the patient's tissue when the distal end of the device 10 contacts the tissue, while the outer member 20 is stiff enough to provide the user with a tactile indication that the end of the device is in surface contact with a patient's tissue and includes adequate hoop strength to maintain the integrity of the lumen 21 when the device is passed through a tortuous path within the patient. The outer member 20 may be made from various flexible, medically acceptable materials such as polyurethane, PEEK, PET, and the like. The material chosen for the outer member 20 should be electrically insulative to prevent significant electrical current to flow therethrough in situations where the plurality of jaws 50 are energized and disposed within the lumen 21 of the outer member 20. Similarly, the material forming the outer member 20 should have a sufficiently low thermal conductivity such that only a minimal amount of heat from the jaws 50 disposed within the lumen 21 of the outer member transfers through the walls of the outer member 20 to damage or burn the patient's tissue. The outer member 20 may be coated with a lubricious material, such as Teflon, to minimize friction between the outer member and the patient's tissue proximate thereto, or the inner surface of a sheath, endoscope or the like that the device 10 may be passed through. The distal end portion of the outer member 20 may be radiopaque or echogenic, or include a radiopaque or echogenic band or similar structure to provide a remote indication of the location of the distal end of the device 10 with respect to the patient's anatomy.

The central member 60 may be an elongate tubular member that defines a lumen 61 therethrough, with the central member 60 being telescopically disposed through the lumen 41 of the intermediate member 40 and movable relative to both the outer member 20 and the intermediate member 40. The central member 60 may be made from various flexible, medically acceptable materials such as polyurethane, PEEK, PET, silicone, and the like. The central member 60 is preferably made from a material that is substantially electrically insulative to prevent a significant amount of electric current to be transferred through the walls of the central member 60 when the distal end portion 62 of the central member is disposed between the plurality of jaws 50 and the jaws 50 are energized with electric current. The central member 60 is also preferably made from a material with a relatively low thermal conductivity to minimize the heat flow through the walls of the central member 60 when disposed between the plurality of jaws 50 in a heated state after tissue cauterization.

In some embodiments, the distal end portion 62 of the central member 60 may be made from a relatively stiffer material than the remainder of the central member 60 with the two portions of the central member 60 fixed or bonded together. As discussed herein, the distal end portion 62 of the central member 60 maintains the jaws 50 in the open position when disposed between the jaws 50, so the distal end portion 62 of the central member 60 should be a material (and with sufficient dimensions) with adequate hoop strength to maintain the jaws 50 in the open position against the inward biasing force of the jaws 50, as well as including sufficiently electrically insulative and with a low thermal conductivity to avoid a biopsy sample disposed within the distal end portion of the central member 60 from being damaged or altered based on the influx of current or heat from the plurality of jaws 50.

The central member 60 may be of a longer length than the outer and intermediate members 20, 40 such that the proximal end portion 64 of the central member 60 extends past the proximal end portions of the outer and intermediate members, which allows the position of the central member 60 to be easily manipulated regardless of the relative telescopic position of each of the outer, intermediate, and central members 20, 40, 60. In some embodiments, the central member 60 may be configured to receive a source of suction thereon, such that the suction forces are directed through the lumen 61 and urge a tissue sample or core disposed within the central member 60 through the lumen 61 of the central member 60 while the device remains installed within the patient. In some embodiments a filter may be disposed between the source of suction and the central member to capture the tissue sample or core urged from the central member. The filter may be removed when the device remains inserted into the patient.

The outer surface of the central member 60 may include a lubricious coating, such as Teflon, that minimizes the friction between the central member 60 and the intermediate member 40 when the two are telescopically slid past each other.

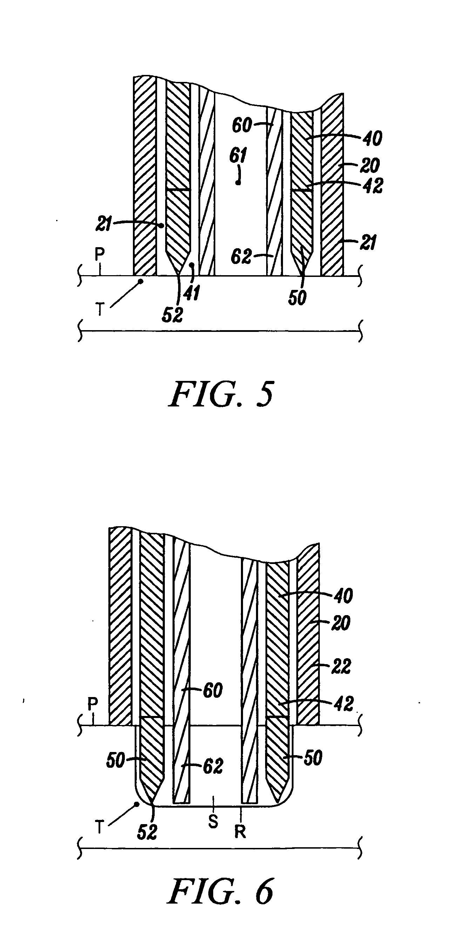

In use and as shown in FIGs. 5-10, the device 10 is inserted into the patient, either through a preexisting lumen or access path of the patient (e.g. inserted through the urethra and then through ureter into the kidney) or the device may be inserted percutaneously into the desired location. In some embodiments, the device 10 may be inserted into the patient through the lumen or working channel of an endoscope, cystoscope, ureteroscope, access sheath, or the like, with such a device previously inserted into the desired location.

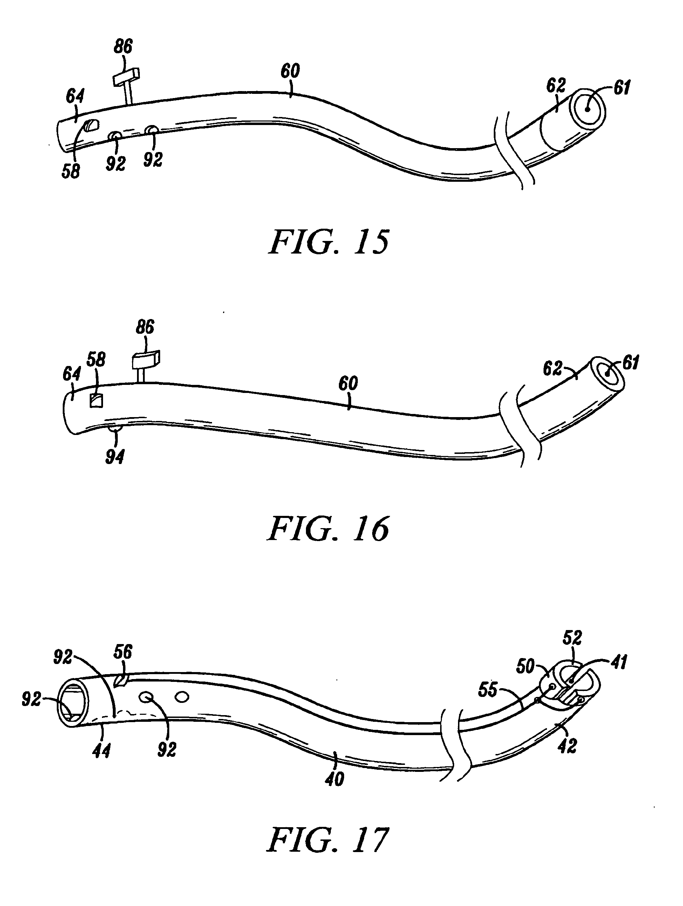

Just prior to insertion, the physician may manipulate the device 10 (using the handle 80 when provided) to align the distal end portions 42, 62 of the intermediate and central members to be flush with or just within the end of the distal end portion 22 of the outer member 20, as shown in FIG. 5.. In embodiments where a detent 94 and one or more recesses 92 (FIGs. 15-17) are disposed on the outer and intermediate members, and/or the intermediate and central member 40, 60 the detents may be aligned within the respective recess to releasably fix the device within this orientation.

The device 10 is guided proximate the location wherein the tissue sample is desired under several aids under the aid of the camera on the endoscope (where provided), under the aid of fluoroscopy, ultrasound or the like when the outer member 20 (or another portion of the device) includes an radiopaque or echogenic portion, and due to the tactile signal received by the physician through the outer member 20 when the device 10 contacts tissue T. When it is desired to obtain a tissue sample S, the jaws 50 of the intermediate member and the distal end portion of the central member 60 are plunged into the tissue T, while the outer member 20 is held fixed, as shown in FIG. 6. The relative motion of the various members may be controlled by the handle 80 with the operators 84, 86 for the intermediate and central members 40, 60 being moved while the handle 80 is maintained stationary.

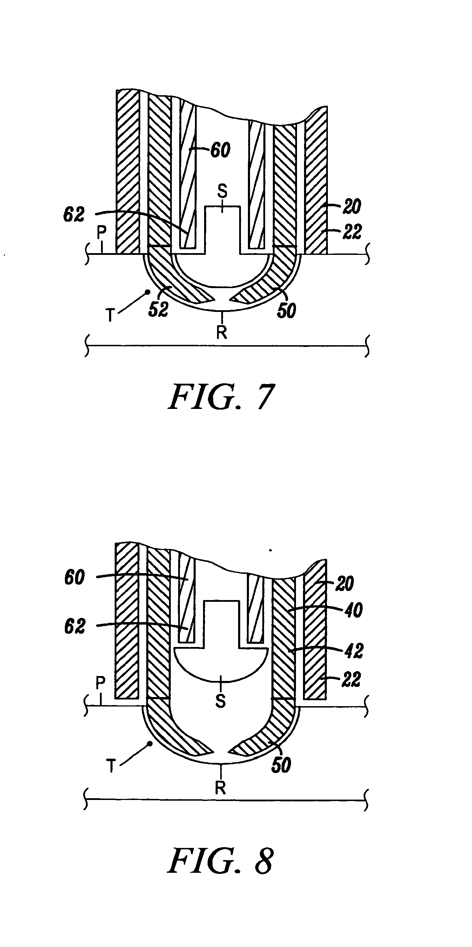

After the jaws 50 and central member 60 is plunged into the tissue T to obtain a suitable biopsy sample, the central member 60 is withdrawn proximally from the jaws 50 using the operator 86 (FIG. 7), when provided. The proximal motion of the central member 60 withdraws the central member 60 from between the jaws 50 which allow the jaws 50 to rotate to their normal biased position. The motion of the jaws 50 within the tissue cuts or slices the tissue T disposed between the jaws S 50 from the remaining tissue due to the relatively sharp edges (or other surface treatments or orientations discussed herein) of the jaws 50. The central member 60 is preferably withdrawn only for the distance required allow the jaws 50 to return to the biased position.

As the tissue sample S is excised or cored from the patient, a portion of the sample S is retained within the lumen 61 of the central member 60. The central member 60 is then further withdrawn proximally to fully remove the tissue sample S from between the jaws 50 As shown in FIG. 8. In devices that include a connection for a flow of suction to the central member 60, suction may be applied to the lumen 61 of the central member 60 to urge the sample S through the central member (and to the filter when provided). The tissue sample S may be withdrawn from the device 10 for analysis as required.

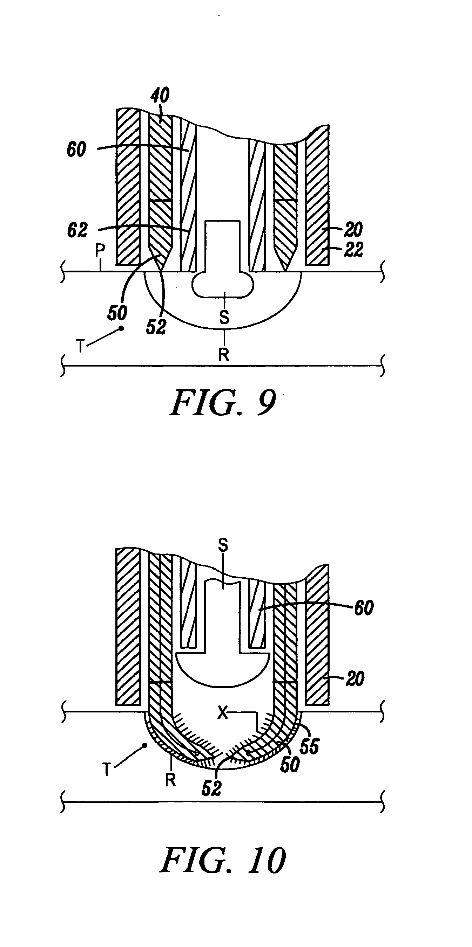

After the central member 60 is adequately withdrawn from between the jaws 50, the device 10 is manipulated to allow electric current (or other type of energy) to flow to the jaws 50, as shown in FIG. 10. As electric current is applied to the jaws 50, the temperature of the jaws 50 rapidly increases to ultimately reach a temperature that will burn or cauterize the tissue R in contact or in close proximity with the jaws 50 (as shown schematically as X in FIG. 10), which may assist or fully eliminate bleeding at the location of the tissue removal R. After the tissue R is adequately cauterized (as may be remotely observed when the device 10 is used in conjunction with an endoscope or the like), the device 10 may be removed from the patient (FIG. 9 showing the device 10 aligned for removal with the outer member 20 disposed around and protecting the intermediate and central members 40, 60), or repositioned for another tissue sample as desired.

In some embodiments, the two or more of the outer, intermediate, and central members 20, 40, 60 may be removably fixed to each other such that the relative telescopic position of each member within the device 10 may be removably fixed with respect to the other members. For example, as shown in FIG. 17, the intermediate member 40 may include one or more recesses 92 disposed within the lumen 41 thereof, preferably located at or near the end of the proximal end potion 44, that are configured to receive a detent 94 that extends from the outer surface of the central member 60. The connection between the detent 94 within the recess 92 removably fixes the position of the central member 60 with respect to the intermediate member 40. In other embodiments shown in FIG. 15, a detent 94 may be provided upon the inner surface of the intermediate member 40, with two or more recesses 92 defined upon the outer surface of the central member 60.

In embodiments where a single recess 92 is provided, the detent 94 and recess 92 may be disposed each on one of the intermediate and central members 40, 60 such that the distal end portion 62 of the central member 60 extends through the plurality of jaws 50 to maintain the jaws 50 in the open position. When it is desired to allow the plurality of jaws 50 to return to the closed position to obtain a tissue sample from the patient, the user may pull the central member 60 proximally while maintaining the intermediate member fixed such that the detent and recess 94, 92 disengage and the distal end portion 62 of the central member 60 is withdrawn from between the plurality of jaws 50.

In other embodiments, two or more recesses may be defined upon one of the intermediate or central members 40, 60. A first of the recesses 92 is aligned to receive the detent 94 when the distal end portion 62 of the central member 60 extends between the plurality of jaws 50, with a second recess 92 is defined provided proximally of the first recess 92 and positioned to receive the detent when the central member is sufficiently withdrawn to allow the plurality of jaws to fully return to the closed position. In some embodiments, the outer member 20 and the intermediate member 40 may additionally include corresponding detent and recesses (similar to those discussed above) which allow the intermediate and outer members 40, 20 to be removably fixed together in a specific telescopic arrangement. For example, the outer member 20 may include a detent and the intermediate member 40 may include one or more recesses 92 (FIG. 17) that are each aligned to fix the end of the distal end potion of the outer member 20 with the edges of the jaws 50 (or with the end of the outer member just extending further than the edges of the jaws 50) such that the members of the device 10 maintain telescopic alignment when the device 10 is being threaded into or out of the patient.

The device 10 may include a handle 80 that allows the user to control the relative telescopic positioning of the outer, intermediate, and central members 20, 40, 60 with respect to each other when the device 10 is inserted into the patient. In some embodiments, the handle 80 may be fixed to one of the three members with two operators 84, 86 provided, each operator fixed to a remaining member. The two operators 84, 86 are each independently movable with respect to the handle 80 and fixed to one of the respective members, such that movement of the operator 84, 86 with respect to the handle 80 causes the member to translate telescopically with respect to the other members.

In some embodiments shown in FIGs. 1-3, the handle 80 is fixed to the proximal end portion of the outer member 20, with a first operator 84 fixed to the proximal end portion 44 of the intermediate member 40 and a second operator 6 fixed to the proximal end portion 64 of the central member 60. The first and second operators 84, 86 may include an interlock 87 that allows the intermediate member 40 and the central member 60 to be advanced distally with respect to the outer member 20 with a single motion by the user. The interlock is easily removable, or may be automatically removed when the user attempts to translate only the central member 60 proximally by manipulating the second operator 86. As shown in FIG. 3, the interlock may be a mechanical device such as a tab 87 disposed on the operator 86 for the central member, which contacts the operator 84 for the intermediate member 40 when the central member 60 is advanced distally within (or with respect to) the outer member 20, by disengages the operator 84 for the intermediate member 40 when the operator 86 and central member 60 are translated proximally with respect to the intermediate member 40. In some embodiments, the handle 80, or various components with in the handle may include markings that provide the user with an indication with the relative position of the various components of the device 10 with respect to each other. The observation of these markings during use, for example markings that show the relative distance between the distal tips of the outer member 20 and the tips of the jaws 50 upon the intermediate member 40, provides the user with an indication of the depth of penetration of the jaws 50 of the device. The known depth of penetration that generates the biopsy sample may provide the physician with a greater understanding of the state of the patient's tissue, especially of multiple biopsy samples are obtained at various depths in neighboring tissue during the same procedure.

The handle 80 may additionally provide a switch, button, breaker, or other control mechanism 89 (FIGs. 1-3) that allows the user to control the application of electrical current (or other energy) to the plurality of jaws 50, from a source of electrical current connected thereto (either remotely through a cord 96 or onboard through batteries 92). In some embodiments, the switch 89 may be disposed in a circuit that includes an interlock that includes a sensor, contact, rela, touch pad or the like 58 (FIGs. 15-16, showing the sensor disposed upon the proximal end portion 64 of the central member 60) that allows current flow to the plurality of jaws 50 when the intermediate member 40 is disposed with respect to the central member 60 (and/or the outer member 20) such that the plurality of jaws 50 extend beyond the distal end portion of the central member 60 and/or the outer member 20, but prevents current flow to the jaws 50 when the jaws are retained with the central member through the jaws and/or the jaws disposed within the lumen of the outer member 20. In some embodiments, the circuit may include a touch pad 58 disposed upon the central member 60 that is configured to contact another touch pad (or similar structure) on the intermediate member 40 when the jaws 50 extend past the central member 60, which causes the circuit to allow current to the jaws 50. In other embodiments, the interlock may be arranged differently to only allow current to the jaws 50 when extended out of the central member 60 and/or outer member 20.

In some embodiments, the device 10 may be configured such that the intermediate member 40 is freely rotatable with respect to the central member 60, such that the plurality of jaws 50 may more effectively and easily cut the biopsy sample or core away from the patient's tissue. The handle 80 of the device may include a dedicated operable surface that allows the user to provide torque to the intermediate member 40 to cause the distal end portion 42 and the plurality of jaws 50 to spin with respect to the central member 60. In other embodiments, the outer, intermediate, and central members 20, 40, 60 may be rotatably fixed together such that rotation of the handle 80 causes similar rotation of the members.

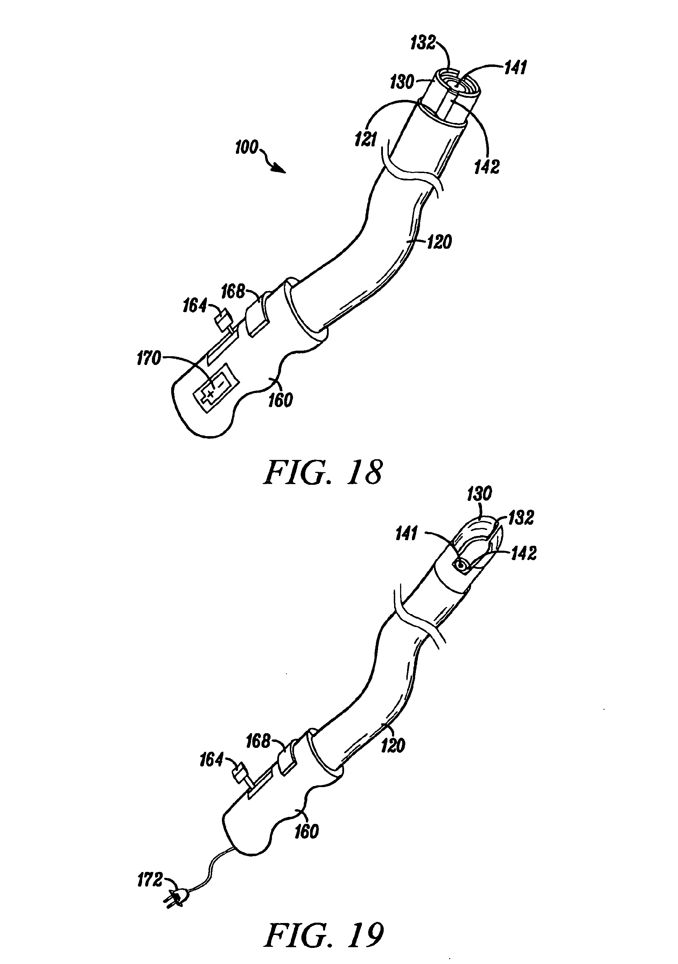

Turning now to FIGs. 18-19, another representative embodiment of a biopsy device 100 is provided. The device 100 is configured to remove a biopsy sample or core from a patient's tissue. The device 100 includes an elongate tubular outer sheath, catheter, cannula, or other tubular member 120 that defines a lumen 121 therethrough, and an inner sheath, catheter, cannula, or other tubular member 140 that defines a lumen 141 therethrough and is telescopically positioned within the lumen 121 of the outer member 120.

The outer member 120 may be formed similarly to the intermediate member 40 discussed above, and may include a distal end portion 122 that includes a plurality of jaws 130 (similar to jaws 50) that are biased into a closed configured (FIG. 19) and can be deformed to an open configuration (FIG. 18) where the jaws 130 are separated from each other. Similarly to the device 10 discussed above, the jaws 130 are urged to the open configuration when a distal end portion 142 of the inner member 140 is disposed between the plurality of jaws 130. The plurality of jaws 130 are aligned in the closed position such that the edges 132 thereof either contact the various edges 132 of the opposing jaws 130, or are in close proximity thereto. The jaws 130 are configured such that a tissue sample or core may be removed from the patient's tissue when the plurality of jaws 130 are plunged into the tissue (generally initially along with the distal end portion of the inner member) and allowed to pivot to the closed position when the inner member 140 is withdrawn proximally from the jaws 130. The tissue sample is removed due to the sharp edges or cutting teeth 132 of the jaws 130 as they move through the tissue to the closed position.

The lumen 141 through the inner member 140 is configured to receive and retain the tissue sample removed from the patient when the jaws 50 are allowed to translate from the open position to the closed position. As with the device 10, the plurality of jaws 130 may be configured to receive electrical current or other energy to significantly increase the temperature of the jaws 130 to cauterize or burn the remaining patient's tissue contacting or closely proximate the jaws 50. The outer member 120 may enclose or support one or more wires therethrough that pass from a source of power to the jaws 130. The outer member 120 may include an insulating (both electrically and heat insulative) coating, such as plastic or rubber, which prevents the electric current or other type of energy passed through or along the outer member 120 or the wires to flow to the tissue of the patient along the length of the device, and to minimize the heat transfer from the heated outer member to the patient.

The device 100 may include a handle 160 (similar in operation and purpose to the handle 80 discussed above) that allows for remote operation of the device 100 within the patient. In some embodiments, the handle 160 is fixed to the outer member 20 with an operator 164 thereon that is fixed to the inner member 140, such that movement of the operator 164 causes the inner member 140 to translate with respect to the outer member 20, based on the direction of motion of the operator. The opposite configuration is equally possible with the handle fixed to the inner member 140 and the operator 164 fixed to and configured to move the outer member 120 with respect to the inner member 140.

In some embodiments, the handle 160 may include a switch, button, or similar operator 168 that selectively allows current flow to the plurality of jaws 130 from the electrical power source (either remote via a cord 172 or via an attached battery 170). The switch 168 may be included in a circuit with an interlock (similar to the circuit discussed above with respect to the device 10) that prevents current flow to the plurality of jaws 130 when the jaws 130 do not extend from the distal end portion 122 of the inner member 120.

In some embodiments, one of the inner or outer members may include one or more recesses (similar to recesses 92 in device 10, above) defined thereon that are engageable by a detent (similar to the detent 94 in device 10, above) disposed on the other of the inner or outer members 120, 140. The detent/recess combination allows the inner and outer members 120, 140 to be removably fixed together. In embodiments with a single recess, the detent and recess may be configured to fix the distal end portion 142 of the inner member 140 within the plurality of jaws 130 to maintain them in the open configuration for insertion into the patient, and to place the jaws 130 in the substantially tubular orientation to allow the jaws 130 to plunge into the patient's tissue to obtain a biopsy sample. In embodiments with two recesses, a second recess may be provided that is selectively engageable with the detent and configured such that the inner member is maintained in a proximal position (not extending through the jaws 130) to allow the biopsy sample to be removed and to maintain the biopsy sample retained within the lumen of the inner member from being affected by the hot jaws 130 during or just after they are electrically heated to cauterize tissue.

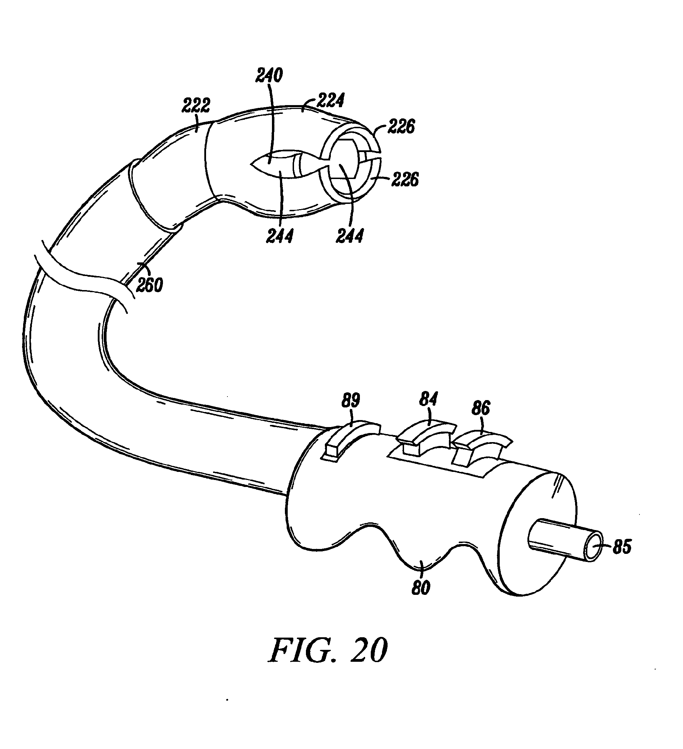

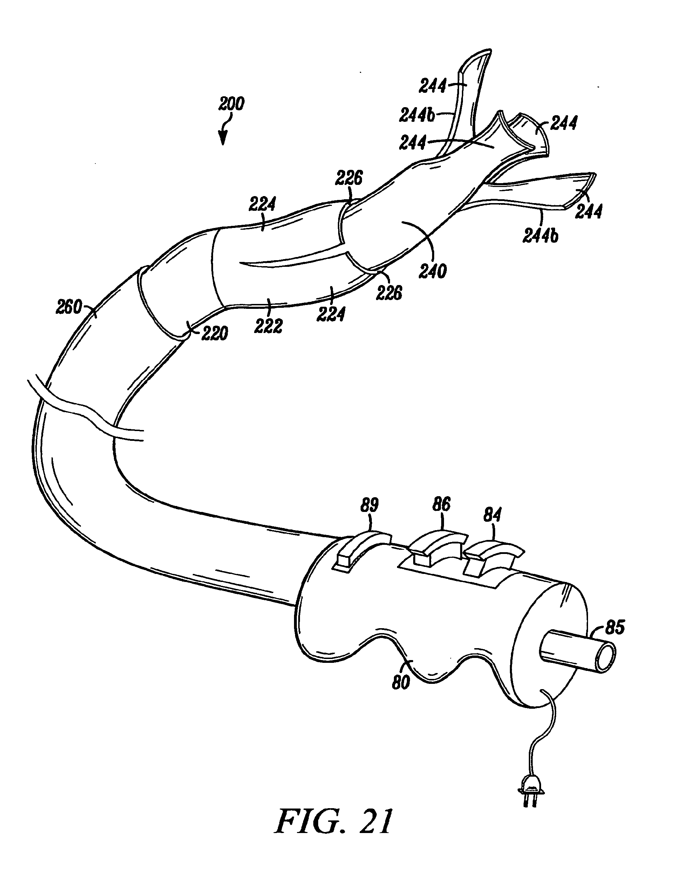

Turning now to FIGs. 20-25, another representative embodiment of a biopsy device 200 is provided. The biopsy device 200 includes an elongate tubular sheath 220 and an elongate inner member 240 that is reciprocatably slidable within a lumen 229 of the sheath 220. The sheath 220 includes a distal end portion 222, a central portion 228, and a proximal end portion (not shown, within sheath 260 in FIGs. 20, 21, similar to proximal end portion 44 of sheath 40, FIG. 17) is mechanically connected to a handle 80. The distal end portion 222 of the sheath 220 may include two or more jaws 224 that are biased into a closed configuration (FIG. 20) where the tips 226 of the two or more jaws are in contact or in close proximity to each other, and can be deformed into an open configuration (FIG. 21) where the distal portion 242 of the inner member 240 may be slid therebetween. In some embodiments, the jaws 224 may be similar to the jaws 50, 130 discussed above.

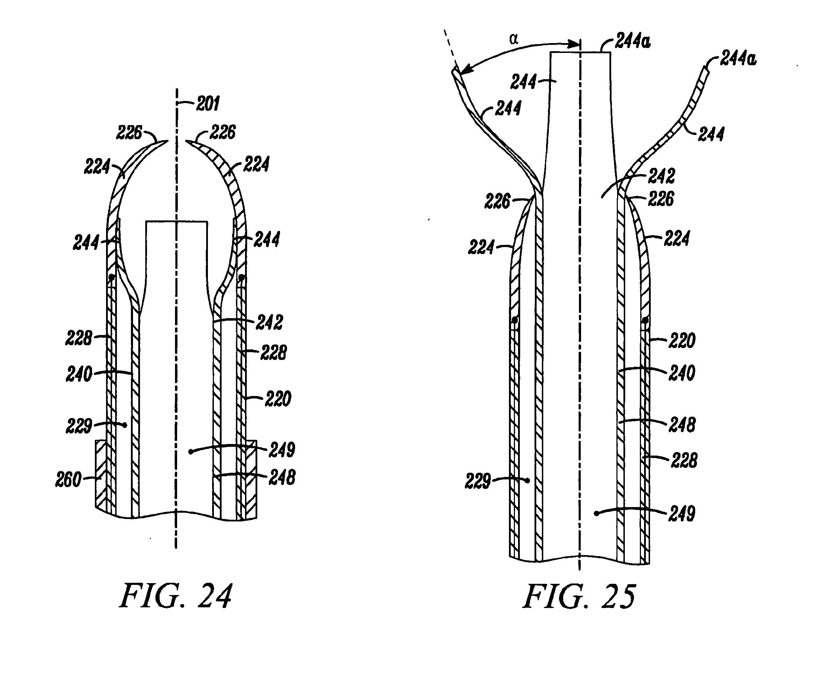

The jaws 224 may each have a sharpened edge or multiple serrations to allow the jaws 224 to sever tissue disposed therebetween when in the closed position, as aided with a rotational force emparted upon the sheath 220. As with the jaws 50, 130 discussed above, the jaws 224 may be configured to receive electrical current or other energy to significantly increase the temperature of the jaws 224 to cauterize or burn the remaining patient's tissue contacting or closely proximate the jaws 224. One or more wires 228 may be disposed upon (or within the walls of) the sheath 220 (FIGs. 24, 25) to transfer electrical current or other energy to the jaws 224 from a remote location of control and generation. The wires 228 may be insulated to provide a high resistance path of current from the wires to the patient.

The jaws 224 may be made from a metal, such as stainless steel or a superelastic alloy, such as Nitinol, such that the jaws 224 are sufficiently flexible and strong to be biased into the closed position while capable of being elastically deformed to the opened configuration, and to allow the jaws 224 to receive and transfer electrical current for cauterizing tissue proximate the tips of the jaws 224. The superelastic alloy may be such that the jaws return to their trained closed position after deformation both at normal body temperature and at elevated temperatures reached just after electrical current is imparted thereto to cauterize tissue. In some embodiments, the sheath 220 may include a flexible portion proximal of the jaws 224, with the flexible portion including an echogenic or radiopaque portion to provide a remote indication of the position of the device within the patient.

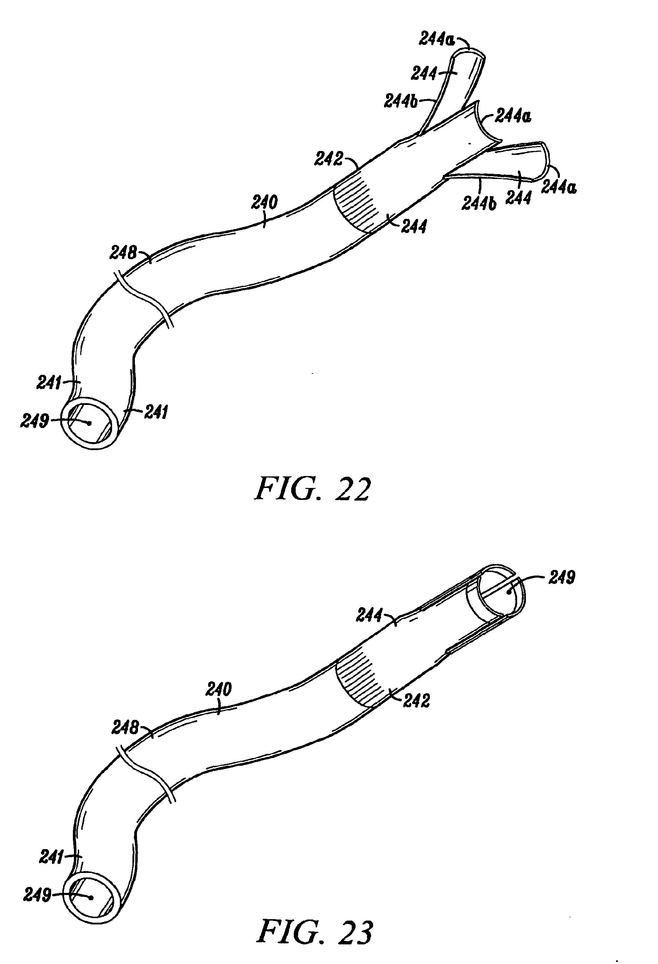

The inner member 240 may include a distal end portion 242 and a proximal end portion 241 (FIGs. 22, 23) and a central portion 248 that connects the distal and proximal end portions 242, 241. The distal end portion 242 may be made from a superelastic alloy, such as Nitinol, or in other embodiments the distal end portion 242 may be stainless steel or other suitable relatively flexible and strong metals. The proximal end portion 241 is fixed to the distal end portion 242 and may be the same material as the distal end portion 242, or in other embodiments may be plastic or another substantially strong and stiff material. In other embodiments, the inner member 240 may be a single monolithic component. The inner member 240 may include a lumen 249 that allows a tissue sample removed from a patient to extend at least partially from the distal end and therethrough.

The distal end portion 242 may include two or more fingers 244 that are biased to extend therefrom at an acute angle α with respect to the longitudinal axis 201 of the inner member 240 (FIG. 25). In other embodiments, there may be three or four, or five fingers 244. In some embodiments as shown in FIG. 22, the fingers 244 may each be biased to extend arcuately from the central portion 248 of the inner member 240, either with a relatively constant curvature or with a curve that changes along its length. In some of these embodiments, the fingers 244 may extend continuously from the central portion 248, while in other embodiments, the fingers 244 may extend discontinuously from the central portion 248 with a junction. In other embodiments, the fingers 244 may be substantially straight along their length, such that the fingers 244 extend discontinuously from the central portion 248. In still other embodiments, the fingers 244 may extend arcuately from the central portion 248 with the distal portions 244a of the fingers 244 being relatively straight. In some embodiments, the fingers 244 may be curved along their width, with the inner surfaces of each finger (244) i.e. the surfaces facing opposing fingers 244 when in the straight configuration (FIG. 23) being in a relatively concave orientation. In some embodiments, the curvature of the fingers 244 may be such that the collective fingers define a substantially circular profile when each finger 244 is in the straight configuration.

The fingers 244 are configured to normally reside in the extended configuration (FIG. 22) and be sufficiently flexible to be urged to a collapsed configuration where each finger 244 extends generally parallel to the longitudinal axis 201 (FIG. 23). As observed in FIGs. 24, the fingers 244 are urged into the straight configuration when the inner member 240 is retracted within the lumen 229 of the sheath 220, such as within the jaws 224, or the central portion 228. When in the collapsed configuration the fingers 244 of the inner member 240 may form a tubular configuration that communicates with the lumen 249, as best shown in FIG. 23, to allow the biopsy sample to be passed or retracted therethrough when removed from the patient. In some embodiments, the lumen 249 of the inner member 240 may be in fluid communication with a source of suction to cause the sample to be vacuum dragged through a suction port 85 in the handle 80, or other suitable portion of the device 200 through the inner member for removal and analysis.

The tips 244a of the fingers 244 may be sharpened to a point or with a beveled tip or edge such that the tips of the fingers 244 puncture, cut, or extend within tissue when urged thereto. In some embodiments, one or both of the side edges 244b of the finger 244 may be sharpened or beveled to allow the fingers themselves to cut the tissue sample disposed within the plurality of fingers from the neighboring tissue for removal of the biopsy sample as the fingers 244 are urged from the extended and angled configuration to the straight configuration. In some embodiments, the inner member 240 may be rotated with respect to the sheath 220, and with respect to the tissue the fingers 244 are extended into, to enhance the cutting of the biopsy sample by the plurality of fingers 244. Accordingly, pivoting of the fingers 244 toward the straight configuration enhances removal of the biopsy sample in conjunction with the pivoting of the jaws 224 of the sheath 220 as the inner member 240 is withdrawn within the lumen 229 of the outer sheath 220, allowing the jaws 224 to pivot to the normal compressed position.

In some embodiments, the device 200 may additionally include an outer sheath 260 (FIGs. 20, 21) that telescopically receives each of the sheath 220 and the inner member 240 within a lumen thereof. The outer sheath 260 may be similar to the outer tubular member 20, discussed above. The outer sheath 260 may be made from plastic or other materials with a substantially low thermal conductivity and a good electrical insulator, to prevent heat from the cauterizing jaws 224 to be conducted to neighboring tissue after use when the device 200 is being removed from the patient, or being repositioned within the patient. The device 200 may include a handle 80, which may be similar to the handle 80 discussed and depicted with respect to the other embodiments above. The handle 80 may be configured with to telescopingly move the sheath 220 and the inner member 240 with respect to each other with operators 84, 86, or other suitable structures, as well as with respect to the outer sheath 260, when provided. The handle 80 may also be configured to provide a suction path therethrough that communicates with the lumen 249 through the inner member 240. The handle 80 may also be configured with an operator 89 to control the flow of current to the jaws 224 for tissue coagulation when desired. In some embodiments, the device may include an interlock (similar to the interlock discussed above) that prevents electrical power flow to the jaws unless the distal end portion 242 and the plurality of fingers 244 are each withdrawn proximally of the plurality of jaws 224 and within the lumen 229 of the sheath 220. In some embodiments, the interlock operates based upon the relative position of the various operators upon the handle 80, while in other embodiments, the interlock operates by sensing the actual relative position of the inner member and fingers with respect to the first member.

While the preferred embodiments of the disclosure have been described, it should be understood that the disclosure is not so limited and modifications may be made without departing from the disclosure. The scope of the invention is defined by the appended claims, and all devices that come within the meaning of the claims, either literally or by equivalence, are intended to be embraced therein.

## Claims

1. A biopsy device (10) comprising:
a first elongate member (40) comprising a distal end portion (42) and a proximal portion (44) with a lumen (41) defined therethrough, the distal end portion comprising two or more, preferably sharpened, metallic jaws (50) that are biased toward each other; and
a second elongate tubular member (60) telescopically and slidably received within the lumen of the first member, the second member comprising a distal end portion (62) with an open distal end, configured to allow tissue to enter the lumen therefrom, the distal end portion of the second member configured to maintain the jaws in a separate configuration when the jaws surround a portion of the second tubular member, **characterized in that** the jaws are configured to receive energy from a remote source to selectively cauterize tissue proximate to the jaws.

2. The biopsy device of claim 1, wherein the second member is rotatable with respect to the first member.

3. The biopsy device of claim 1, further comprising a tubular third member (20) defining a distal end portion (41) and a proximal end portion, with the third member telescopically and slidably disposed around the first member.

4. The biopsy device of any of the preceding claims, further comprising a handle (80) receiving the proximal end portions of each of the first and second members, wherein the handle further comprises a first operator (84) configured to translate the first member (40) with respect to the third member, and a second operator (86) configured to translate the second member (60) with respect to the first member.

5. The biopsy device of any one of the preceding claims, further comprising an energy transmission line (55) connected with the jaws and the proximal end portion of the first member, the transmission line configured to receive energy from an energy source.

6. The biopsy device of any one of the preceding claims, wherein the distal end portion of second member is formed from one or more substantially electrically insulating materials.

7. The biopsy device of any of the preceeding claims, further comprising an interlock (87) preventing the application of energy to the jaws unless the distal end portion of the second member is at least partially telescopically withdrawn proximally of the jaws.

8. The biopsy device of any of the preceeding claims, wherein the device is configured to be extended through a working channel of an endoscope, a ureteroscope, a cystoscope, or an access sheath.

9. The biopsy device of any one of the preceding claims, further comprising a detent (94) defined upon one of the first and second members and a plurality of recesses (92) defined along the length of the other of the first and second members, the detent engageable with one of the plurality of recesses to maintain the first and second members longitudinally and rotationally fixed to each other.

10. The biopsy device of any one of the preceding claims, wherein the second member is configured to receive a source of suction through the lumen of the second member to urge foreign material proximally through the lumen of the second member.

11. The biopsy device of any of the preceeding claims, wherein the second member further comprises a plurality of fingers (244) disposed upon a distal end thereof, wherein the plurality of fingers are each biased to an extended position extending at an oblique angle with respect to a longitudinal axis of the device when the distal end portion of the second member extends distally of the jaws, and configured to each be urged into a collapsed position relatively parallel with the longitudinal axis when the distal end portion of the second member is withdrawn between the jaws and within the lumen of the first member.

12. The biopsy device of claim 11, wherein a distal end (244a) of each of the plurality of fingers includes a sharpened tip or a sharpened edge.

13. The biopsy device of claim 11 or 12, wherein the plurality of fingers combine to form a substantial tubular geometry when each of the plurality of fingers are disposed in the collapsed position.

14. The biopsy device of any of claims 11-13, wherein the plurality of fingers are configured to collect and sever a sample of material when the pressed into the material in the extended configuration and urged to the compressed configuration by withdrawing the second member proximally with respect to the first member.

15. The biopsy device of any of the preceeding claims, wherein the combined distal end portions of the device are sufficiently flexible to be at aligned at an oblique angle to a longitudinal axis through the proximal end portion of the first member.

## Patentansprüche

1. Biopsievorrichtung (10), umfassend:
ein erstes langgestrecktes Element (40) mit einem distalen Endabschnitt (42) und einem proximalen Endabschnitt (44) mit einem dort hindurch definierten Lumen (41), wobei der distale Endabschnitt zwei oder mehr vorzugsweise geschärfte Metallbacken (50) umfasst, die zueinander vorgespannt sind; und
ein zweites langgestrecktes röhrenförmiges Element (60), das teleskopisch und gleitend in dem Lumen des ersten Elements aufgenommen ist, wobei das zweite Element einen distalen Endabschnitt (62) mit einem offenen distalen Ende aufweist, der ausgelegt ist, das Eindringen von Gewebe in das Lumen von dort zu gestatten, wobei der distale Endabschnitt des zweiten Elements ausgelegt ist, die Backen in einer getrennten Konfiguration zu halten, wenn die Backen einen Abschnitt des zweiten röhrenförmigen Elements umgeben, **dadurch gekennzeichnet, dass** die Backen zur Aufnahme von Energie von einer entfernten Quelle zur selektiven Kauterisation von Gewebe neben den Backen ausgelegt sind.

2. Biopsievorrichtung nach Anspruch 1, worin das zweite Element bezüglich des ersten Elements drehbar ist.

3. Biopsievorrichtung nach Anspruch 1, ferner ein röhrenförmiges drittes Element (20) umfassend, das einen distalen Endabschnitt (22) und einen proximalen Endabschnitt definiert, wobei das dritte Element teleskopisch und gleitend um das erste Element angeordnet ist.

4. Biopsievorrichtung nach einem der vorhergehenden Ansprüche, ferner einen Griff (80) umfassend, der die proximalen Endabschnitte jedes der ersten und zweiten Elemente aufnimmt, worin der Griff ferner ein erstes Betätigungselement (84) zur Versetzung des ersten Elements (40) bezüglich des dritten Elements und ein zweites Betätigungselement (86) zur Versetzung des zweiten Elements (60) bezüglich des ersten Elements umfasst.

5. Biopsievorrichtung nach einem der vorhergehenden Ansprüche, ferner eine Energieübertragungsleitung (55) umfassend, die mit den Backen und dem proximalen Endabschnitt des ersten Elements verbunden ist, wobei die Übertragungsleitung zur Aufnahme von Energie von einer Energiequelle ausgelegt ist.

6. Biopsievorrichtung nach einem der vorhergehenden Ansprüche, worin der distale Endabschnitt des zweiten Elements aus einem oder mehreren im Wesentlichen elektrisch isolierenden Materialien geformt ist.

7. Biopsievorrichtung nach einem der vorhergehenden Ansprüche, ferner eine Sperre (87) umfassend, die das Aufbringen von Energie auf die Backen verhindert, wenn der distale Endabschnitt des zweiten Elements nicht zumindest teilweise teleskopisch proximal von den Backen zurückgezogen wurde.

8. Biopsievorrichtung nach einem der vorhergehenden Ansprüche, worin die Vorrichtung ausgelegt ist, durch einen Arbeitskanal eines Endoskops, eines Uteroskops, eines Zystoskops oder einer Zugangsschleuse ausgezogen zu werden.

9. Biopsievorrichtung nach einem der vorhergehenden Ansprüche, ferner eine Arretierung (94), die auf einem der ersten und zweiten Elemente definiert ist, und eine Vielzahl von Vertiefungen (92) umfassend, die entlang der Länge des anderen der ersten und zweiten Elemente definiert ist, wobei die Arretierung mit einer der Vielzahl von Vertiefungen in Eingriff gebracht werden kann, um die ersten und zweiten Elemente längs und drehend aneinander fixiert zu halten.

10. Biopsievorrichtung nach einem der vorhergehenden Ansprüche, worin das zweite Element zur Aufnahme einer Saugquelle durch das Lumen des zweiten Elements ausgelegt ist, um Fremdmaterial proximal durch das Lumen des zweiten Elements zu drücken.

11. Biopsievorrichtung nach einem der vorhergehenden Ansprüche, worin das zweite Element ferner eine Vielzahl von Fingern (244) umfasst, die auf einem distalen Ende davon angeordnet sind, worin die Vielzahl von Fingern jeweils in eine ausgezogene Stellung vorgespannt sind, die sich in einem schiefen Winkel bezüglich einer Längsachse der Vorrichtung erstreckt, wenn sich der distale Endabschnitt des zweiten Elements distal von den Backen erstreckt, und die ausgelegt sind, jeweils in eine kollabierte Stellung relativ parallel mit der Längsachse gedrückt zu werden, wenn der distale Endabschnitt des zweiten Elements zwischen den Backen und in dem Lumen des ersten Elements zurückgezogen wird.

12. Biopsievorrichtung nach Anspruch 11, worin ein distales Ende (244a) jeder der Vielzahl von Fingern eine geschärfte Spitze oder eine geschärfte Kante aufweist.

13. Biopsievorrichtung nach Anspruch 11 oder 12, worin die Vielzahl von Fingern gemeinsam eine im Wesentlichen röhrenförmige Geometrie bilden, wenn jeder der Vielzahl von Fingern in der kollabierten Stellung angeordnet ist.

14. Biopsievorrichtung nach einem der Ansprüche 11-13, worin die Vielzahl von Fingern ausgelegt sind, eine Materialprobe zu sammeln und abzutrennen, wenn sie in das Material in der ausgezogenen Stellung gedrückt werden und in die komprimierte Konfiguration gedrückt werden, indem das zweite Element proximal bezüglich des ersten Elements zurückgezogen wird.

15. Biopsievorrichtung nach einem der vorhergehenden Ansprüche, worin die kombinierten distalen Endabschnitte der Vorrichtung ausreichend flexibel sind, um in einem schiefen Winkel zu einer Längsachse durch den proximalen Endabschnitt des ersten Elements ausgerichtet zu werden.

## Revendications

1. Dispositif (10) de biopsie comportant :
un premier élément (40) allongé comportant une partie (42) d'extrémité distale et une partie (44) proximale présentant une lumière (41) délimitée à travers ce dernier, la partie d'extrémité distale comportant deux mâchoires (50) métalliques ou plus, de préférence acérées, qui sont sollicitées l'une vers l'autre ; et
un deuxième élément (60) tubulaire allongé reçu de manière télescopique et coulissante à l'intérieur de la lumière du premier élément, le deuxième élément comportant une partie (62) d'extrémité distale présentant une extrémité distale ouverte, configurée pour permettre à un tissu d'entrer dans la lumière depuis cette dernière, la partie d'extrémité distale du deuxième élément étant configurée pour maintenir les mâchoires dans une configuration séparée lorsque les mâchoires entourent une partie du deuxième élément tubulaire, **caractérisé en ce que** les mâchoires sont configurées pour recevoir de l'énergie provenant d'une source éloignée afin de cautériser sélectivement un tissu à proximité des mâchoires.

2. Dispositif de biopsie selon la revendication 1, le deuxième élément étant rotatif par rapport au premier élément.

3. Dispositif de biopsie selon la revendication 1, comportant en outre un troisième élément (20) tubulaire délimitant une partie (22) d'extrémité distale et une partie d'extrémité proximale, avec le troisième élément disposé de manière télescopique et coulissante autour du premier élément.

4. Dispositif de biopsie selon l'une quelconque des revendications précédentes, comportant en outre une poignée (80) qui reçoit les parties d'extrémité proximale de chacun des premier et deuxième éléments, la poignée comportant en outre un premier opérateur (84) configuré pour déplacer en translation le premier élément (40) par rapport au troisième élément, et un deuxième opérateur (86) configuré pour déplacer en translation le deuxième élément (60) par rapport au premier élément.

5. Dispositif de biopsie selon l'une quelconque des revendications précédentes, comportant en outre une ligne (55) de transmission d'énergie reliée aux mâchoires et à la partie d'extrémité proximale du premier élément, la ligne de transmission étant configurée pour recevoir de l'énergie provenant d'une source d'énergie.

6. Dispositif de biopsie selon l'une quelconque des revendications précédentes, la partie d'extrémité distale du deuxième élément étant formée dans une ou plusieurs matières sensiblement électro-isolantes.

7. Dispositif de biopsie selon l'une quelconque des revendications précédentes, comportant en outre un verrouillage réciproque (87) empêchant l'application d'énergie sur les mâchoires à moins que la partie d'extrémité distale du deuxième élément soit au moins partiellement rentrée télescopiquement de manière proximale par rapport aux mâchoires.

8. Dispositif de biopsie selon l'une quelconque des revendications précédentes, le dispositif étant configuré pour être passé dans un canal de travail d'un endoscope, d'un urétéroscope, d'un cystoscope, ou d'une gaine d'accès.

9. Dispositif de biopsie selon l'une quelconque des revendications précédentes, comportant en outre une détente (94) délimitée sur l'un ou l'autre des premier et deuxième éléments et plusieurs renfoncements (92) délimités sur la longueur de l'un ou l'autre des premier et deuxième éléments, la détente pouvant se mettre en prise avec un renfoncement desdits plusieurs renfoncements pour maintenir les premier et deuxième éléments fixés l'un par rapport à l'autre longitudinalement et rotationnellement.

10. Dispositif de biopsie selon l'une quelconque des revendications précédentes, le deuxième élément étant configuré pour recevoir une source d'aspiration dans la lumière du deuxième élément pour forcer une matière étrangère proximalement dans la lumière du deuxième élément.

11. Dispositif de biopsie selon l'une quelconque des revendications précédentes, le deuxième élément comportant en outre plusieurs doigts (244) disposés sur une extrémité distale de ce dernier, les doigts desdits plusieurs doigts étant sollicités vers une position déployée en formant un angle oblique par rapport à un axe longitudinal du dispositif lorsque la partie d'extrémité distale du deuxième élément s'étend distalement par rapport aux mâchoires, et étant configurés pour être chacun sollicité dans une position repliée relativement parallèle à l'axe longitudinal lorsque la partie d'extrémité distale du deuxième élément est rentrée entre les mâchoires et à l'intérieur de la lumière du premier élément.

12. Dispositif de biopsie selon la revendication 11, une extrémité (244a) distale de chacun desdits plusieurs doigts comprenant une pointe acérée ou un bord acéré.

13. Dispositif de biopsie selon la revendication 11 ou 12, les doigts desdits plusieurs doigts se combinant pour former une géométrie sensiblement tubulaire lorsque chaque doigt desdits plusieurs doigts est disposé dans la position repliée.

14. Dispositif de biopsie selon l'une quelconque des revendications 11 à 13, lesdits plusieurs doigts étant configurés pour recueillir et sectionner un échantillon de matière lorsqu'ils sont enfoncés dans la matière dans la configuration déployée et étant amenés vers la configuration comprimée en rentrant le deuxième élément proximalement par rapport au premier élément.

15. Dispositif de biopsie selon l'une quelconque des revendications précédentes, les parties d'extrémité distale combinées du dispositif étant suffisamment souples pour être alignées en formant un angle oblique par rapport à un axe longitudinal dans la partie d'extrémité proximale du premier élément.
